# EUROPÄISCHE PATENTANMELDUNG

(11) **EP 1 369 410 A1**
(43) Veröffentlichungstag der Anmeldung: **10.12.2003**
(21) Anmeldenummer: 03011881.4
(22) Anmeldetag: 27.05.2003
(51) Int. Cl.: C07C 67/317, C07D 333/24, C07D 333/28, C07C 51/377, C07C 231/18, C07C 253/30

(54) **Verfahren zur stereoselektiven Reduktion von 4-Aryl-4-oxobutansäure-derivaten**

(30) Priorität: 07.06.2002 DE 10225352
(71) Anmelder: BAYER AG, 51368 Leverkusen (DE)
(72) Erfinder: Militzer, H.-Christian, Dr., 51519 Odenthal (DE); Bosch, Boris, Dr., 50668 Köln (DE); Eckert, Markus, Dr., Shanghai 200001 (CN); Meseguer, Benjamin, Dr., 43005 Tarragona (ES)

(57) **Zusammenfassung**

Die vorliegende Erfindung betrifft ein Verfahren zur Herstellung stereoisomerenangereicherter 4-Aryl-4-hydroxybutansäurederivate durch Reduktion von 4-Aryl-4-ketobutansäurederivaten in Gegenwart von Ruthenium enthaltenden Katalysatoren, von mindestens einem Amin, und von Ameisensäure, Formiaten oder Mischungen davon.

## Beschreibung

Die vorliegende Erfindung betrifft ein Verfahren zur Herstellung stereoisomerenangereicherter 4-Aryl-4-hydroxybutansäurederivate durch Reduktion von 4-Aryl-4-ketobutansäurederivaten in Gegenwart von Ruthenium enthaltenden Katalysatoren.

Stereoisomerenangereicherte 4-Hydroxybutansäurederivate sind wertvolle Zwischenprodukte zum Beispiel bei der Herstellung von flüssigkristallinen Verbindungen, Agrochemikalien und Pharmazeutika.

Die katalytische Reduktion von Ketonen zu stereoisomerenangereicherten sekundären Alkoholen ist prinzipiell bekannt. Als Reduktionsmittel kommen dabei üblicherweise molekularer Wasserstoff oder bei sogenannten Transferhydrierungen organische Wasserstoffdonoren wie zum Beispiel Ameisensäure oder Isopropanol in Frage.

Noyori et al. beschreiben in EP-A 744 401 die enantioselektive Wasserstoffhydrierung von γ-Ketoestem und nachfolgende Cyclisierung zu den entsprechenden Butyrolactonen in Gegenwart von chiralen Rutheniumkomplexen. Nachteilig sind hier die extrem langen Reaktionszeiten von mehreren Tagen.

Ein Vorteil von Transferhydrierungen besteht darin, dass die sicherheitstechnischen Vorkehrungen die mit der Handhabung hochentzündlichen molekularen Wasserstoffs unter Druck getroffen werden müssen, entfallen. Weiterhin kann in der Regel bei Umgebungsdruck gearbeitet werden.

Eine zusammenfassende Darstellung über Transferhydrierungen als Methode zur katalytischen Reduktion von Ketonen geben z.B. Zassinovich et al. in Chem. Rev. 1992, 92, 1051-1069 und Noyori et al. in Acc. Chem. Res. 1997, 30, 97-102 sowie Wills et al. in Tetrahedron, Asymmetry, 1999, 2045.

Noyori et al. (JACS 1996, 118, 2521-2522, Acc. Chem. Res. 1997, 30, 97-102) beschreiben die Verwendung von Rutheniumkomplexen als Katalysatoren und Triethylamin/Ameisensäure als Wasserstoffdonor-Mischung für die enantioselektive Reduktion einfacher Ketone.

Es bestand jedoch weiterhin das Bedürfnis, ein effizientes Verfahren bereitzustellen, das die Herstellung von stereoisomerenangereicherten 4-Aryl-4-hydroxybutansäurederivaten aus 4-Aryl-4-oxobutansäurederivaten erlaubt.

Es wurde nun ein Verfahren zur Herstellung von stereoisomerenangereicherten 4-Aryl-4-hydroxybutansäurederivaten gefunden, das dadurch gekennzeichnet ist, dass
a) Verbindungen der Formel (I)

   Ar-CO-CH₂CH₂W (I),

   in der
   - Ar: für einen mono-, bi- oder tricyclischen aromatischen Rest mit insgesamt 5 bis 18 Ringatomen steht, wobei pro Cyclus keines, eines zwei oder drei Ringatome ausgewählt aus der Gruppe Sauerstoff, Schwefel und Stickstoff vorhanden sein können, und wobei der mono-, bi- oder tricyclische aromatische Rest gegebenenfalls einfach oder mehrfach substituiert ist und
   - W: für C(O)YR¹ₙ steht, wobei Y = für Sauerstoff steht und n = 1 ist oder Y für Stickstoff steht und n = 2 ist, oder
   - W: für CN steht und
   - R¹: jeweils unabhängig voneinander für Wasserstoff, C₁-C₂₀-Alkyl, C₄-C₁₄-Aryl oder C₅-C₁₅-Arylalkyl oder im Falle, dass Y für Stickstoff steht, die beiden Reste R¹ zusammen für C₃-C₁₂ Alkylen stehen,
b) in Gegenwart eines Ruthenium enthaltenden Katalysators und
c) in Gegenwart von mindestens einem Amin, das zumindest teilweise in protonierter Form vorliegt,
d) mit Ameisensäure, Formiaten oder Mischungen davon
e) gegebenenfalls in Gegenwart von organischem Lösungsmittel
umgesetzt werden.

Es sei darauf hingewiesen, dass vom Umfang der Erfindung auch beliebige Kombinationen der genannten Bereiche und Vorzugsbereiche für jedes Merkmal (z.B. Parameter oder Strukturmerkmal) umfasst sind.

Verbindungen der Formel (I) sind z.B. in prinzipiell bekannter Weise in einem Schritt durch Friedel-Crafts-Acylierung aus dem entsprechenden Aromaten und Bemsteinsäureanhydrid in Gegenwart einer Lewis-Säure wie Aluminiumchlorid oder Zinnchlorid erhältlich (siehe auch J. March in Advanced Organic Chemistry, 4^{th} Ed., John Wiley and Sons, 1992, Seite 541).

Stereoisomerenangereicherte (enantiomerenangereicherte, diastereomerenangereicherte) 4-Aryl-4-hydroxybutansäurederivate im Sinne der Erfindung bedeuten stereoisomerenreine (enantiomerenreine bzw. diastereomerenreine) 4-Aryl-4-hydroxybutansäurederivate oder Mischungen von stereoisomeren (enantiomeren bzw. diastereomeren) 4-Aryl-4-hydroxybutansäurederivaten, in denen ein Stereoisomeres (Enantiomeres bzw. Diastereomeres) in einem größeren absoluten Anteil, bevorzugt 70 bis 100 Mol-% und ganz besonders bevorzugt 85 bis 100 Mol-%, vorliegt, als ein anderes Diastereomeres bzw. als das andere Enantiomere.

**Alkyl** steht im Rahmen der Erfindung jeweils unabhängig für einen geradkettigen oder cyclischen, unabhängig davon verzweigten oder unverzweigten Alkyl-Rest, der durch Cᵢ-C₄-Alkoxy-Reste weiter substituiert sein kann. Gleiches gilt für den Alkylenteil eines Arylalkyl-Restes.

Beispielsweise steht im Rahmen der Erfindung C₁-C₄-Alkyl für Methyl, Ethyl, 2-Ethoxyethyl, n-Propyl, Isopropyl, n-Butyl und tert.-Butyl, C₁-C_{g}-Alkyl darüber hinaus beispielsweise für n-Pentyl, Cyclohexyl, n-Hexyl, n-Heptyl, n-Octyl oder iso-Octyl, C₁-C₁₂ -Alkyl, weiter darüber hinaus z. B. für Norbomyl, n-Decyl und n-Dodecyl und C1-C₂₀ noch weiter darüber hinaus für n-Hexadecyl und n-Octadecyl.

**Aryl** steht im Rahmen der Erfindung beispielsweise und bevorzugt für carbocyclische aromatische Reste oder heteroaromatische Reste, die keines, ein, zwei oder drei Heteroatome pro Cyclus, im gesamten heteroaromatische Rest mindestens jedoch ein Heteroatom enthalten, das ausgewählt ist aus der Gruppe Stickstoff, Schwefel oder Sauerstoff.

Weiterhin können die carbocyclischen aromatischen Reste oder heteroaromatischen Reste mit bis zu fünf Substituenten pro Cyclus substituiert sein, die jeweils unabhängig voneinander beispielsweise und bevorzugt ausgewählt sind aus der Gruppe Hydroxy, C₁-C₁₂-Alkyl, Cyano, COOH, COOM, wobei M für ein Alkalimetallion oder ein halbes Äquivalent eines Erdalkalimetallions steht, COO-(C₁-C₁₂-Alkyl), COO-(C₄-C₁₀-Aryl), CO-(C₁-C₁₂-Alkyl), CO-(C₄-C₁₀-Aryl), O-(C₁-C₁₂-Alkyl), O-(C₄-C₁₀-Aryl, N(C₁-C₁₂-Alkyl)₂, NH-(C₁-C₁₂-Alkyl), Fluor, Chlor, Brom, C₁-C₁₂-Fluoralkyl, wobei Fluoralkyl für einen einfach mehrfach oder vollständig durch Fluor substituierten Alkylrest gemäß obiger Definition steht, CONH₂, CONH-(C₁-C₁₂-Alkyl), NHCOO-(C₁-C₁₂-Alkyl). Gleiches gilt für den Arylteil eines Arylalkyl-Restes.

In Formel (I) steht Ar bevorzugt für einen mono- oder bicyclischen aromatischen Rest mit insgesamt 5 bis 12 Ringatomen, wobei pro Cyclus keines, eines oder zwei Ringatome ausgewählt aus der Gruppe Sauerstoff, Schwefel und Stickstoff vorhanden sein können und wobei der mono- oder bicyclische aromatische Rest keinen, einen, zwei oder drei Reste pro Cyclus trägt, die jeweils unabhängig voneinander ausgewählt sind aus der Gruppe Hydroxy, C₁-C₁₂-Alkyl, Cyano, COOH, COOM, COO-(C₁-C₁₂-Alkyl), COO-(C₄-C₁₀-Aryl), CO-(C₁-C₁₂-Alkyl), CO-(C₄-C₁₀-Aryl), O-(C₁ -C₁₂-Alkyl), (C₁-C₁₂-Alkyl)-O-(C₁-C₁₂-Alkyl), (C₄-C₁₀-Aryl)-O-(C₁ -C₁₂-Alkyl), O-(C₄-C₁₀-Aryl), O-CO-(C₄-C₁₀-Aryl), O-CO-(C₁-C₁₂-Alkyl), OCOO-(C₁-C₁₂-Alkyl), N-(C₁-C₁₂-Alkyl)₂, NH-(C₁-C₁₂-Alkyl), N(C₄-C₁₀-Aryl)₂, NH-(C₄-C₁₀-Aryl), Fluor, Chlor, Brom, Iod, NO₂, SO₃H, SO₃M, SO₂(C₁-C₁₂-Alkyl), SO(C₁-C₁₂-Alkyl), C₁-C₁₂-Fluoralkyl, wobei Fluoralkyl für einen einfach mehrfach oder vollständig durch Fluor substituierten Alkylrest gemäß obiger Definition steht, NHCO-(C₁-C₁₂-Alkyl), CONH₂, CONH-(C₁-C₁₂-Alkyl), NHCOO-(C₁-C₁₂-Alkyl), PO(C₄-C₁₀-Aryl)₂, PO(C₁-C₁₂-Alkyl)₂, PO₃H₂, PO₃M_{2,} PO₃HM, PO(O(C₁-C₁₂-Alkyl)₂, wobei M jeweils für ein Alkalimetallion oder ein halbes Äquivalent eines Erdalkalimetallions steht.

In Formel (I) steht Ar bevorzugt für Phenyl, 1- oder 2-Naphthyl, 1-,2-, 3- oder 4-Fluorenyl und 1-,2- oder 5-Anthracenyl, Phenanthrenyl, Heteroaryl besonders bevorzugt für 2- oder 3-Thiophenyl, 2- oder 3-Furanyl, 2- oder 3-Pyrrolyl, 3- oder 4-Pyrazolyl, 1-, 2-, oder 4-Thiazolyl, 1-, 2-, oder 4-Oxazolyl, 2-, 4- oder 5-Imidazolyl, 2-, 3-, oder 4-Pyridyl, 2- oder 3-Pyrazinyl, 2-, 4-, oder 5-Pyrimidyl, 3-, 4-, 5- oder 6-Pyridazinyl, 2- oder 3-Indolyl, 3-Indazolyl, Indazolyl, 2- oder 3-Benzofüranyl, 2- oder 3-Benzothiophen-yl, 2-, 3- oder 4-Chinolinyl, Isochinolinyl 2-, 4-, 6- oder 7-Pteridinyl oder 2-, 3-, 4-, 5-, 6-, 8-, 9- oder 10-Phenanthrenyl wobei jeder der genannten Reste keinen, einen oder zwei Reste pro Cyclus trägt, die jeweils unabhängig voneinander ausgewählt sind aus der Gruppe C₁-C₄-Alkyl, Cyano, COO-(C₁-C₄-Alkyl), O-(C₁-C₄-Alkyl), N(C₁-C₄-Alkyl)₂, NH-(C₁-C₄-Alkyl), Fluor, Chlor, Brom oder C₁-C₄-Fluoralkyl wie zum Beispiel Trifluormethyl, 2,2,2-Trifluorethyl oder Pentafluorethyl.

Ganz besonders bevorzugt steht in Formel (I) Ar für 2-Thiophen-yl.

Bevorzugt steht W in Formel (I) für COOR¹, wobei R¹ für Wasserstoff oder C₁-Cg-Alkyl steht.

Bevorzugt steht in Formel (I) R¹ für C₁-C₁₂-Alkyl, Phenyl, o-, m-, p-Tolyl, p-Nitrophenyl oder Benzyl.

Besonders bevorzugt steht in Formel (I) R¹ für Methyl, Ethyl, 2-Ethoxyethyl, n-Propyl, Isopropyl, n-Butyl, tert.-Butyl, n-Pentyl, Cyclohexyl und n-Hexyl sowie für Trifluormethyl, Chlormethyl, Benzyl und Phenyl. sowie für 1,5-Pentylen, 1,4-Butylen und 1,3-Propylen.

Ganz besonders bevorzugte Verbindungen der Formel (I) sind
Methyl-4-oxo-4-(phenyl)butanoat, Methyl-4-oxo-4-[(2-, 3- oder 4-methyl-; 2-, 3- oder 4-ethyl-, 2-, 3- oder 4-ísopropyl; 2-, 3- oder 4-tert.butyl-; 2-, 3- oder 4-methoxy; 2-, 3- oder 4- trifluormethyl; 2-, 3- oder 4-trifluormethoxy-; 2-, 3- oder 4-brom-; 2-, 3- oder 4-chlor-; 2-, 3- oder 4-fluor-; 2-, 3- oder 4-cyano-; 2-, 3- oder 4-methoxycarbonyl-; 2-, 3- oder 4-dimethylamino-; 2-, 3- oder 4-diethylamino-; 2-, 3- oder 4-dimethylamino-; 2-, 3- oder 4-methylamino-)phenyl]-butanoat, Ethyl-4-oxo-4-(phenyl)butanoat, Ethyl-4-oxo-4-[(2-, 3- oder 4-methyl-; 2-, 3- oder 4-ethyl-, 2-, 3- oder 4-ísopropyl; 2-, 3- oder 4-tert.butyl-; 2-, 3- oder 4-methoxy; 2-, 3- oder 4- trifluormethyl; 2-, 3- oder 4-trifluormethoxy-; 2-, 3- oder 4-brom-; 2-, 3- oder 4-chlor-; 2-, 3- oder 4-fluor-; 2-, 3- oder 4-cyano-; 2-, 3- oder 4-methoxycarbonyl-; 2-, 3- oder 4-dimethylamino-; 2-, 3- oder 4-diethylamino-; 2-, 3- oder 4-dimethylamino-; 2-, 3- oder 4-methylamino-)phenyl]-butanoat, Isopropyl-4-oxo-4-(phenyl)butanoat, Isopropyl-4-oxo-4-[(2-, 3- oder 4-methyl-; 2-, 3- oder 4-ethyl-, 2-, 3- oder 4-isopropyl; 2-, 3- oder 4-tert.butyl-; 2-, 3- oder 4-methoxy; 2-, 3- oder 4- trifluormethyl; 2-, 3- oder 4-trifluormethoxy-; 2-, 3- oder 4-brom-; 2-, 3- oder 4-chlor-; 2-, 3- oder 4-fluor-; 2-, 3- oder 4-cyano-; 2-, 3- oder 4-methoxycarbonyl-; 2-, 3- oder 4-dimethylamino-; 2-, 3- oder 4-diethylamino-; 2-, 3- oder 4-dimethylamino-; 2-, 3- oder 4-methylamino-)phenyl]-butanoat,

Tert.-butyl-4-oxo-4-(phenyl)butanoat, Tert.-butyl-4-oxo-4-[(2-, 3- oder 4-methyl-; 2-, 3- oder 4-ethyl-, 2-, 3- oder 4-isopropyl; 2-, 3- oder 4-tert.butyl-; 2-, 3- oder 4-methoxy; 2-, 3- oder 4- trifluormethyl; 2-, 3- oder 4-trifluormethoxy-; 2-, 3- oder 4-brom-; 2-, 3- oder 4-chlor-; 2-, 3- oder 4-fluor-; 2-, 3- oder 4-cyano-; 2-, 3- oder 4-methoxycarbonyl-; 2-, 3- oder 4-dimethylamino-; 2-, 3- oder 4-diethylamino-; 2-, 3- oder 4-dimethylamino-; 2-, 3- oder 4-methylamino-)phenyl]-butanoat,

2-Ethylhexyl-4-oxo-4-(phenyl)butanoat, 2-Ethylhexyl-4-oxo-4-[(2-, 3- oder 4-methyl-; 2-, 3- oder 4-ethyl-, 2-, 3- oder 4-isopropyl; 2-, 3- oder 4-tert.butyl-; 2-, 3- oder 4-methoxy; 2-, 3- oder 4- trifluormethyl; 2-, 3- oder 4-trifluormethoxy-; 2-, 3- oder 4-brom-; 2-, 3- oder 4-chlor-; 2-, 3- oder 4-fluor-; 2-, 3- oder 4-cyano-; 2-, 3- oder 4-methoxycarbonyl-; 2-, 3- oder 4-dimethylamino-; 2-, 3- oder 4-diethylamino-; 2-, 3- oder 4-dimethylamino-; 2-, 3- oder 4-methylamino-)phenyl]-butanoat, Methyl-4-oxo-4-(2- oder 3-thiophenyl-; 2- oder 3-furanyl-; 2- oder 3-pyrrolyl-; 3- oder 4-pyrazolyl-; 1-, 2- oder 4-thiazolyl-; 1-, 2- oder 4-oxazolyl-; 2-, 4- oder 5-imidazolyl-; 2-, 3- oder 4-pyridyl-; 2- oder 3-pyrazinyl-; 2-, 4- oder 5-pyrimidyl; 3-, 4-, 5- oder 6-pyridazinyl-; 2- oder 3-indolyl-; 3-indazolyl-; indazolyl-; 2- oder 3-benzofüranyl-; 2- oder 3-benzothiophenyl-; 2-, 3- oder 4-chinolinyl- oder isochinolinyl)-butanoat, Ethyl-4-oxo-4-(2- oder 3-thiophenyl-; 2- oder 3-furanyl-; 2- oder 3-pyrrolyl-; 3- oder 4-pyrazolyl-; 1-, 2- oder 4-thiazolyl-; 1-, 2- oder 4-oxazolyl-; 2-, 4- oder 5-imidazolyl-; 2-, 3- oder 4-pyridyl-; 2- oder 3-pyrazinyl-; 2-, 4- oder 5-pyrimidyl; 3-, 4-, 5- oder 6-pyridazinyl-; 2- oder 3-indolyl-; 3-indazolyl-; indazolyl-; 2- oder 3-benzofuranyl-; 2- oder 3-benzothiophen-yl-; 2-, 3- oder 4-chinolinyl- oder isochinolinyl)-butanoat, Isopropyl-4-oxo-4-(2- oder 3-thiophenyl-; 2- oder 3-füranyl-; 2- oder 3-pyrrolyl-; 3- oder 4-pyrazolyl-; 1-, 2- oder 4-thiazolyl-; 1-, 2- oder 4-oxazolyl-; 2-, 4- oder 5-imidazolyl-; 2-, 3- oder 4-pyridyl-; 2- oder 3-pyrazinyl-; 2-, 4- oder 5-pyrimidyl; 3-, 4-, 5- oder 6-pyridazinyl-; 2- oder 3-indolyl-; 3-indazolyl-; indazolyl-; 2- oder 3-benzofuranyl-; 2- oder 3-benzothiophenyl-; 2-, 3- oder 4-chinolinyl- oder isochinolinyl)-butanoat,
Tert.-butyl-4-oxo-4-(2- oder 3-thiophenyl-; 2- oder 3-furanyl-; 2- oder 3-pyrrolyl-; 3- oder 4-pyrazolyl-; 1-, 2- oder 4-thiazolyl-; 1-, 2- oder 4-oxazolyl-; 2-, 4- oder 5-imidazolyl-; 2-, 3- oder 4-pyridyl-; 2- oder 3-pyrazinyl-; 2-, 4- oder 5-pyrimidyl; 3-, 4-, 5- oder 6-pyridazinyl-; 2- oder 3-indolyl-; 3-indazolyl-; indazolyl-; 2- oder 3-benzofüranyl-; 2- oder 3-benzothiophenyl-; 2-, 3- oder 4-chinolinyl- oder isochinolinyl)-butanoat,
2-Ethylhexyl-4-oxo-4-(2- oder 3-thiophenyl-; 2- oder 3-füranyl-; 2- oder 3-pyrrolyl-; 3- oder 4-pyrazolyl-; 1-, 2- oder 4-thiazolyl-; 1-, 2- oder 4-oxazolyl-; 2-, 4- oder 5-imidazolyl-; 2-, 3- oder 4-pyridyl-; 2- oder 3-pyrazinyl-; 2-, 4- oder 5-pyrimidyl; 3-, 4-, 5- oder 6-pyridazinyl-; 2- oder 3-indolyl-; 3-indazolyl-; indazolyl-; 2- oder 3-benzofüranyl-; 2- oder 3-benzothiophenyl-; 2-, 3- oder 4-chinolinyl- oder isochinolinyl)-butanoat,
wobei Methyl-4-oxo-4-(2-thiophen-yl)-butanoat und Ethyl-4-oxo-4-(2-thiophen-yl)-butanoat noch weiter bevorzugt sind.

Das erfindungsgemäße Verfahren wird in Gegenwart eines Ruthenium enthaltenden Katalysators durchgeführt.

Beispielsweise und bevorzugt werden solche Katalysatoren verwendet, die Rutheniumkomplexe enthalten. Bevorzugte Rutheniumkomplexe sind solche, die durch Umsetzung von Verbindungen der Formel (II) mit Verbindungen der Formel (III) erhältlich sind, oder Komplexe der Formel (IV). Besonders bevorzugt werden solche Rutheniumkomplexe eingesetzt, die durch Umsetzung von Verbindungen der Formel (II) mit Verbindungen der Formel (III) erhältlich sind. In einer bevorzugten Ausführungsform beträgt dabei das molare Verhältnis von Verbindungen der Formel (III) und Verbindungen der Formel (II) 2:1 bis 3:1 besonders bevorzugt 2,01:1 bis 2,4:1.

Vorteilhafterweise werden Verbindungen der Formel (III) und Verbindungen der Formel (II) gemischt und die Mischung in organischem Lösungsmittel aufgenommen. Die resultierende Mischung kann weiterhin vorteilhafterweise vor Zugabe zur Reaktionsmischung mit einer Base, bevorzugt einem Amin versetzt und beispielsweise und bevorzugt 10 bis 30 min gerührt werden, wobei die molare Menge des Amins beispielsweise und bevorzugt 1:1 bis 3:1, besonders bevorzugt 1:1 bis 2:1 bezogen auf Verbindungen der Formel (III) beträgt.

Für organische Lösungsmittel und Amine gelten die gleichen Angaben und Vorzugsbereiche, die weiter unten detailliert beschrieben werden.

In den Verbindungen der Formel (II)

[RuX₂(Aren)]₂ (II)

steht
- Aren: für eine koordinierte aromatische Verbindung mit 6 bis 12 Ringkohlenstoffatomen, die weiterhin mit bis zu 6 Resten substituiert sein kann, die jeweils unabhängig voneinander ausgewählt sind aus der Gruppe C₁-C₈-Alkyl, Benzyl und Phenyl und
- X: beispielsweise und bevorzugt für Chlor, Brom oder Iod besonders bevorzugt für Chlor.

Bevorzugt steht Aren für Benzol oder Naphthalin, das mit bis zu 6 Resten substituiert sein kann, die jeweils unabhängig voneinander ausgewählt sind aus der Gruppe Methyl, Ethyl, n-Propyl, Isopropyl und tert.-Butyl.

Bevorzugt steht Aren für Mesitylen, Cumol oder Benzol.

Besonders bevorzugte Verbindungen der Formel (II) sind Benzoldichlororuthenium-Dimer, Mesitylendichlororuthenium-Dimer und Cumoldichlororuthenium-Dimer, wobei Cumoldichlororuthenium-Dimer noch weiter bevorzugt ist.

In der Formel (III) 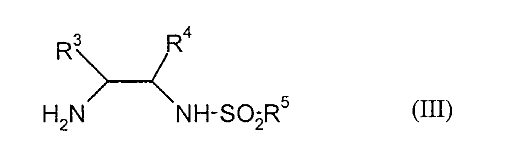 stehen
- R³ und R⁴: jeweils unabhängig voneinander beispielsweise für C₁-C₂₀-Alkyl, C₄-C₁₅-Aryl oder C₅-C₁₆-Arylalkyl oder R³ und R⁴ zusammen für einen geradkettigen oder verzweigten C₃-C₁₂-Alkylen-Rest, und
- R⁵: für C₁-C₂₀-Alkyl, C₁-C₂₀-Fluoralkyl oder C₄-C₁₅-Aryl.

Bevorzugt stehen R³ und R⁴ jeweils identisch für Phenyl oder zusammen für geradkettiges C₃-C₈-Alkylen wie zum Beispiel 1,3-Pentylen oder 1,4-Butylen, besonders bevorzugt stehen R³ und R⁴ jeweils identisch für Phenyl.
- R⁵: steht bevorzugt für C₁-C₄-Alkyl, C₁-C₄-Fluoralkyl, Phenyl oder Naphthyl, das mit keinem, einem, zwei, drei vier oder fünf Resten weiter substituiert sein kann, die ausgewählt sind aus der Gruppe C₁-C₄-Alkyl, C₁-C₄-Alkoxy, C₁-C₄-Fluoralkyl, Fluor und Chlor.
- R⁵: steht besonders bevorzugt für Methyl, Trifluormethyl, Pentafluorethyl, Nonafluorbutyl, Phenyl, p-Tolyl, p-Ethylphenyl, p-Anisyl, p-Ethoxyphenyl, p-Chlorphenyl, 2,4,6-Trimethylphenyl, 2,4,6-Triisopropylphenyl, p-Fluorphenyl, Pentafluorphenyl, und Naphthyl.
- R⁵: steht besonders bevorzugt für p-Tolyl, Phenyl, Naphtyl.
- R⁵: steht ganz besonders bevorzugt für p-Tolyl.

Bevorzugt weisen die Verbindungen der Formel (III) eine Stereoisomerenreinheit von 90 % oder mehr, besonders bevorzugt von 95 % oder mehr und ganz besonders bevorzugt von 98,5 % oder mehr auf.

Als Verbindungen der Formel (III) seien genannt:
N-[(1R,2R) und (1S,2S)-2-amino-1,2-diphenylethyl]-p-tolylsulfonamid, N-[(1R,2R) und (1S,2S)-2-amino-1,2-diphenylethyl]-o-tolylsulfonamid, N-[(1R,2R) und (1S,2S)-2-amino-1,2-diphenylethyl]-m-tolylsulfonamid, N-[(1R,2R) und (1S,2S)-2-amino-1,2-diphenylethyl]-phenylsulfonamid, N-[(1R,2R) und (1S,2S)-2-amino-1,2-diphenylethyl] -4-ethylphenylsulfonamid, N-[(1R,2R) und (1S,2S)-2-amino-1,2-diphenylethyl]-3-ethylphenylsulfonamid, N-[(1R,2R) und (1S,2S)-2-amino-1,2-diphenylethyl]-2-ethylphenylsulfonamid, N-[(1R,2R) und (1S.2S)-2-amino-1,2-diphenylethyl]-2,4,6-trimethylphenylsulfonamid, N-[(1R,2R) und (1S,2S)-2-amino-1,2-diphenylethyl]-2,4,6-triisopropylphenylsulfonamid, N-[(1R,2R) und (1S,2S)-2-amino-1,2-diphenylethyl]-4-chlorphenylsulfonamid, N-[(1R,2R) und (1S,2S)-2-amino-1,2-diphenylethyl]-3-chlorphenylsulfonamid, N-[(1R,2R) und (1S,2S)-2-amino-1,2-diphenylethyl]-2-chlorphenylsulfonamid, N-[(1R,2R) und (1S,2S)-2-amino-1,2-diphenylethyl]-4-fluorphenylsulfonamid, N-[(1R,2R) und (1S,2S)-2-amino-1,2-diphenylethyl]-3-fluorphenylsulfonamid, N-[(1R,2R) und (1S,2S)-2-amino-1,2-diphenylethyl] -2-fluorphenylsulfonamid, N-[(1R,2R) und (1S,2S)-2-amino-1,2-diphenylethyl]-4-methoxyphenylsulfonamid, N-[(1R,2R) und (1S,2S)-2-amino-1,2-diphenylethyl]-3-methoxyphenylsulfonamid, N-[(1R,2R) und (1S,2S)-2-amino-1,2-diphenylethyl]-2-methoxyphenylsulfonamid, N-[(1R,2R) und (1S,2S)-2-amino-1,2-diphenylethyl]- 1 -naphtylsulfonamid, N-[(1R,2R) und (1S,2S)-2-amino-1,2-diphenylethyl]-2-naphtylsulfonamid, N-[(1R,2R) und (1S,2S)-2-amino-1,2-diphenylethyl]-pentafluorphenylsulfonamid, N-[(1R,2R) und (1S,2S)-2-ammo-1,2-diphenylethyl]-methansulfonamid, N-[(1R,2R) und (1S,2S)-2-amino-1,2-diphenylethyl]-trifluormethansulfonamid, N-[(1R,2R) und (1S,2S)-2-aminocyclohexyl]-p-tolylsulfonamid, N-[(1R,2R) und (1 S,25)-2-aminocyclohexyl]-o-tolylsulfonamid, N-[(1R,2R) und (1S,2S)-2-aminocyclohexyl]-m-tolylsulfonamid, N-[(1R,2R) und (1S,2S)-2-aminocyclohexyl]-phenylsulfonamid, N-[(1R,2R) und (1S,2S)-2-aminocyclohexyl]-4-ethylphenylsulfonamid, N-[(1R,2R) und (1S,2S)-2-aminocyclohexyl]-3-ethylphenylsulfonamid, N-[(1R,2R) und (1S,2S)-2-aminocyclohexyl]-2-ethylphenylsulfonamid, N-[(1R,2R) und (1S,2S)-2-aminocyclohexyl]-2,4,6-trimethylphenylsulfonamid, N-[(1R,2R) und (1S,2S)-2-aminocyclohexyl]-2,4,6-triisopropylphenylsulfonamid, N-[(1R,2R) und (1S,2S)-2-aminocyclohexyl]-4-chlorphenylsulfonamid, N-[(1R,2R) und (1S,2S)-2-aminocyclohexyl]-3-chlorphenylsulfonamid, N-[(1R,2R) und (1S,2S)-2-aminocyclohexyl]-2-chlorphenylsulfonamid, N-[(1R,2R) und (1S,2S)-2-aminocyclohexyl]-4-fluorphenylsulfonamid, N-[(1R,2R) und (1S,2S)-2-aminocyclohexyl]-3-fluorphenylsulfonamid, N-[(1R,2R) und (1S,2S)-2-aminocyclohexyl]-2-fluorphenylsulfonamid, N-[(1R,2R) und (1S,2S)-2-aminocyclohexyl]-4-methoxyphenylsulfonamid, N-[(1R,2R) und (1S,2S)-2-aminocyclohexyl]-3-methoxyphenylsulfonamid, N-[(1R,2R) und (1S,2S)-2-aminocyclohexyl]-2-methoxyphenylsulfonamid, N-[(1R,2R) und (1S,2S)-2-aminocyclohexyl]-1-naphtylsulfonamid, N-[(1R,2R) und (1S,2S)-2-aminocyclohexyl]-2-naphtylsulfonamid, N-[(1R,2R) und (1S,2S)-2-aminocyclohexyl]-pentafluorphenylsulfonamid, N-[(1R,2R) und (1S,2S)-2-aminocyclohexyl]-methansulfonamid, N-[(1R,2R) und (1S,2S)-2-aminocyclohexyl]-trifluormethansulfonamid, N-[(1R,2R) und (1S,2S)-2-aminocyclopentyl]-p-tolylsulfonamid, N-[(1R,2R) und (1S,2S)-2-aminocyclopentyl]-o-tolylsulfonamid, N-[(1R,2R) und (1S,2S)-2-aminocyclopentyl]-m-tolylsulfonamid, N-[(1R,2R) und (1S,2S)-2-aminocyclopentyl]-phenylsulfonamid, N-[(1R,2R) und (1S,2S)-2-aminocyclopentyl]-4-ethylphenylsulfonamid, N-[(1R,2R) und (1S,2S)-2-aminocyclopentyl]-3-ethylphenylsulfonamid, N-[(1R,2R) und (1S,2S)-2-aminocyclopentyl] -2-ethylphenylsulfonamid, N-[(1R,2R) und (1S,2S)-2-aminocyclopentyl]-2,4,6-trimethylphenylsulfonamid, N-[(1R,2R) und (1S,2S)-2-aminocyclopentyl]-2,4,6-triisopropylphenylsulfonamid, N-[(1R,2R) und (1S,2S)-2-aminocyclopentyl]-4-chlorphenylsulfonamid, N-[(1R,2R) und (1S,2S)-2-aminocyclopentyl]-3-chlorphenylsulfonamid, N-[(1R,2R) und (1S,2S)-2-aminocyclopentyl]-2-chlorphenylsulfonamid, N-[(1R,2R) und (1S,2S)-2-aminocyclopentyl]-4-fluorphenylsulfonamid, N-[(1R,2R) und (1S,2S)-2-aminocyclopentyl]-3-fluorphenylsulfonamid, N-[(1R,2R) und (1S,2S)-2-aminocyclopentyl]-2-fluorphenylsulfonamid, N-[(1R,2R) und (1S,2S)-2-aminocyclopentyl]-4-methoxyphenylsulfonamid, N-[(1R,2R) und (1S,2S)-2-aminocyclopentyl]-3-methoxyphenylsulfonamid, N-[(1R,2R) und (1S,2S)-2-aminocyclopentyl]-2-methoxyphenylsulfonamid, N-[(1R,2R) und (1S,2S)-2-aminocyclopentyl]-1-naphtylsulfonamid, N-[(1R,2R) und (1S,2S)-2-aminocyclopentyl]-2-naphtylsulfonamid, N-[(1R,2R) und (1S,2S)-2-aminocyclopentyl]-pentafluorphenylsulfonamid, N-[(1R,2R) und (1S,2S)-2-aminocyclopentyl]-methansulfonamid sowie N-[(1R,2R) und (1S,2S)-2-aminocyclopentyl]-trifluormethansulfonamid.

In der Formel (IV)

[RuX₂(Aren){(III)}] (IV)

besitzen Aren und X jeweils die unter Formel (II) angegebene Bedeutung und Vorzugsbereiche, (III) steht in der Formel (IV) für Verbindungen der Formel (III) mit den dort angegebenen Bedeutung und Vorzugsbereichen.

Als Verbindungen der Formel (IV) seien genannt:
[N-[(1R,2R und 1S,2S)-2-(amino-κN)-1,2-diphenylethyl]-p-tolylsulfonamidato-κN]chloro[(η⁶)-cumol]-ruthenium(II),
[N-[(1R,2R und 1S,2S)-2-(amino-κN)-1,2-diphenylethyl]-phenylsulfonamidato-κN]chloro [η⁶)-cumol]-ruthenium(II),
[N-[(1R,2R und 1S,2S)-2-(amino-κN)-1,2-diphenylethyl]-2,4,6-trimethylphenylsulfonamidato-κN] chloro [(η⁶)-cumol] -ruthenium(II),
[N-[(1R,2R und 1S,2S)-2-(amino-κN)-1,2-diphenylethyl]-4-chlorphenylsulfonamidato-κN]chloro[(η⁶)-cumol]-ruthenium(II),
[N-[(1R,2R und 1S,2S)-2-(amino-κN)-1,2-diphenylethyl]-4-fluorphenylsulfonamidato-κN]chloro[(η⁶)-cumol]-ruthenium(II)
[N-[(1R,2R und 1 S,2S)-2-(amino-κN)- 1,2-diphenylethyl]-4-methoxyphenylsulfonamidato-κN]chloro[(η⁶)-cumol]-ruthenium(II)
[N-[(1R,2R und 1S,2S)-2-(amino-κN)-1,2-diphenylethyl]-1-naphtylsulfonamidato-κN]chloro[(η⁶)-cumol]-ruthenium(II)
[N-[(1R,2R und 1S,2S)-2-(amino-κN)-1,2-diphenylethyl]-pentafluorphenylsulfonamidato-κN]chloro[(η⁶)-cumol]-ruthenium(II)
[N-[(1R,2R und 1S,2S)-2-(amino-κN)- 1,2-diphenylethyl]-methansulfonamidatoκN] chloro [(η⁶)-cumol]-ruthenium(II)
[N-[(1R,2R und 1S,2S)-2-(amino-κN)-1,2-diphenylethyl]-trifluormethansulfonamidato-κN]chloro[(η⁶)-cumol]-ruthenium(II)
[N-[(1R,2R und 1S,2S)-2-(amino-κN)-1,2-diphenylethyl]-p-tolylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-p-tolylsulfonamidato-κN]chloro [(η⁶)-benzol]-ruthenium(II)
[N-[(1R,2R und 1S,2S)-2-(amino-κN)-1,2-diphenylethyl]-2,4,6-trimethylphenylsulfonamidato-κN]chloro[(η⁶)-benzol]-ruthenium(II)
[N-[(1R,2R und 1S,2S)-2-(amino-κN)- 1,2-diphenylethyl]-1-naphtylsulfonamidato-κN]chloro [(η⁶)-benzol]-ruthenium(II)
[N-[(1R,2R und 1S,2S)-2-(amino-κN)-1,2-diphenylethyl]-pentafluorphenylsulfonamidato-κN]chloro[(η⁶)-benzol]-ruthenium(II)
[N-[(1R,2R und 1S,2S)-2-(amino-κN)-cyclohexyl]-p-tolylsulfonamidato-κN]chloro-[η⁶)-cumol]-ruthenium(II)
[N-[(1R,2R und 1S,2S)-2-(amino-κN)-cyclohexyl]-phenylsulfonamidato-κN]chloro-[(η⁶)-cumol]-ruthenium(II)
[N-[(1R,2R und 1S,2S)-2-(amino-κN)-cyclohexyl]-2,4,6-trimethylphenylsulfonamidato-κN]chloro[(η⁶)-cumol]-ruthenium(II)
[N-[(1R,2R und 1S,2S)-2-(amino-κN)-cyclohexyl]-4-chlorphenylsulfonamidato-κN]chloro[(η⁶)-cumol]-ruthenium(II)
[N-[(1R,2R und 1S,2S)-2-(amino-κN)-cyclohexyl]-4-fluorphenylsulfonamidato-κN]chloro[(η⁶)-cumol]-ruthenium(II)
[N-[(1R,2R und 1S,2S)-2-(amino-κN)-cyclohexyl]-4-methoxyphenylsulfonamidato-κN] chloro [(η⁶)-cumol]-ruthenium(II)
[N-[(1R,2R und 1S,2S)-2-(amino-κN)-cyclohexyl]-1-naphtylsulfonamidato-κN]chloro(η⁶)-cumol]-ruthenium(II)
[N-[(1R,2R und 1S,2S)-2-(amino-κN)-cyclohexyl]-methansulfonamidato-κN] chloro-[(η⁶)-cumol]-ruthemum(II)
[N-[(1R,2R und 1S,2S)-2-(amino-κN)-cyclohexyl]-trifluormethansulfonamidato-1(N]chloro[η⁶)-cumol]-ruthenium(II)
[N-[(1R,2R und 1S,2S)-2-(amino-κN)-cyclohexyl]-p-tolylsulfonamidato-κN]chloro-[(η⁶)-benzol]-ruthenium(II)
[N-[(1R,2R und 1S,2S)-2-(amino-κN)-cyclohexyl]-2,4,6-trimethylphenylsulfonamidato-κN]chloro[(η⁶)-benzol]-ruthenium(II) und
[N-[(1R,2R und 1S,2S)-2-(amino-κN)-cyclohexyl]-p-tolylsulfonamidato-κN]chloro-[(η⁶)-1,3,5-trimethylbenzol]-ruthenium(II)

Besonders bevorzugte Katalysatoren in Sinne der Erfindung sind solche, die Rutheniumkomplexe enthalten, die durch Umsetzung von S,S- oder R,R-N-p-Toluolsulfonyl- 1 ,2-diphenylethylendiamin und Cumoldichlororuthenium-Dimer erhältlich sind.

Das erfindungsgemäße Verfahren wird in Gegenwart von mindestens einem Amin, bevorzugt einem Amin durchgeführt, das zumindest teilweise in protonierter Form vorliegt.

Weiterhin werden für das erfindungsgemäße Verfahren Ameisensäure, Formiate oder Mischungen davon eingesetzt.

Bevorzugt werden Mischungen von Ameisensäure mit Aminen eingesetzt. Auf diese Weise bilden sich zumindest teilweise die entsprechenden Ammoniumformiate, die analog eingesetzt werden können.

Als Amine sind insbesondere solche der Formel (V) geeignet,

NR⁶R⁷R⁸ (V)

in der
- R⁶, R⁷ und R⁸: jeweils unabhängig voneinander für Wasserstoff, C₁-Cg-Alkyl oder Benzyl stehen.

Besonders bevorzugte Amine sind Ammoniak und solche der Formel (V) in der R⁶, R⁷ und R⁸ jeweils unabhängig voneinander für C₁-C_{g}-Alkyl oder Benzyl stehen.

Besonders bevorzugte Amine sind solche der Formel (V) in der R⁶, R⁷ und R⁸ jeweils identisch für Ethyl, n-Butyl oder n-Hexyl stehen, wobei der Einsatz von Triethylamin noch weiter bevorzugt ist.

Das molare Verhältnis von Ameisensäure zu Amin kann beispielsweise 1:1 bis 3:1 betragen, bevorzugt ist ein Verhältnis von 1,01:1 bis 1,5:1.

Das molare Verhältnis an Ameisensäure bezogen auf eingesetztes Substrat kann beispielsweise 1:1 bis 3:1 betragen, bevorzugt sind 1:1 bis 1,5 : 1, besonders bevorzugt 1,02 : 1 bis 1,1 : 1.

Das erfindungsgemäße Verfahren kann in Gegenwart oder Abwesenheit, bevorzugt in Gegenwart von organischem Lösungsmittel durchgeführt werden.

Geeignete organische Lösungsmittel sind beispielsweise:

Amide wie z.B. Dimethylformamid, N-Methylpyrrolidinon, gegebenenfalls halogenierte aliphatische oder araliphatische Lösungsmittel mit bis zu 16 Kohlenstoffatomen wie z.B. Toluol, o-, m-, p-Xylol, Chloroform, Dichlormethan, Chlorbenzol, die isomeren Dichlorbenzole, Fluorbenzol, Nitrile wie zum Beispiel Acetonitril, Benzonitril, o -Dimethylsulfoxid oder Mischungen davon.

Bevorzugte Lösungsmittel sind Acetonitril, N-Methylpyrrolidinon, Chloroform, Dichlormethan, Chlorbenzol, die isomeren Dichlorbenzole, Fluorbenzol oder Mischungen davon, besonders bevorzugt sind Dichlormethan, Acetonitril, N-Methylpyrrolidon oder Mischungen davon.

Die Reaktionstemperatur kann beispielsweise -10 bis 150°C betragen, bevorzugt sind 20 bis 100°C, besonders bevorzugt 20 bis 80°C.

Die Reaktionszeiten liegen beispielsweise zwischen 0,5 h bis 48 h, bevorzugt zwischen 6 und 24 h.

Die molare Menge an Ruthenium kann beispielsweise 0,01 bis 1,0 Mol-%, bezogen auf das eingesetzte Substrat betragen, bevorzugt sind 0,02 bis 0,2 Mol-%, ganz besonders bevorzugt 0,02 bis 0,1 Mol-%.

Es ist vorteilhaft, wenn auch nicht obligatorisch, die Reaktion in einer im Wesentlichen sauerstofffreien Atmosphäre durchzuführen. Im Wesentlichen sauerstofffrei bedeutet dabei beispielsweise einen Gehalt von 0 bis 1 Vol-%, bevorzugt 0 bis 0,1 Vol-% Sauerstoff.

Die Reaktion kann durch die Entfernung von Kohlendioxid, welches während der Reaktion freigesetzt wird, beschleunigt werden. Vorteilhaft und daher von der Erfindung umfasst ist intensives Rühren des Reaktionsgemisches mit einer durchschnittlichen Rührerdrehzahl von beispielsweise 100 bis 3 000 min⁻¹, bevorzugt 500 bis 1 500 min⁻¹. Alternativ oder in Ergänzung dazu kann die Entfernung von Kohlendioxid durch Durchleiten oder Überleiten eines inerten Gasstroms durch oder über die Reaktionsmischung unterstützt werden. Geeignete Gase sind beispielsweise Stickstoff, Edelgase wie z.B. Argon oder Mischungen davon.

Auf erfindungsgemäße Weise erhält man stereoisomerenangereicherte 4-Aryl-4-hydroxy-butansäurederivate der Formel (VI),

Ar-CH(OH)-CH₂CH₂W (VI),

in der Ar und W die gleichen Bedeutungen und Vorzugsbereiche besitzen, die unter der Formel (I) genannt wurden.
Dabei sind je nach Wahl der Konfiguration der Liganden die an 4-Position S- oder R-konfigurierten Produkte erhältlich.

Eine besonders bevorzugte Ausführung des erfindungsgemäßen Prozesses wird nachfolgend beschrieben, ohne jedoch einschränkend zu sein.

In einem Rührkessel wird eine 1:1 Mischung (molar) aus Ameisensäure und Triethylamin durch einfaches Mischen hergestellt und das 4-Aryl-4-keto-butansäurederivat äquimolar oder in geringem Unterschuss zu dieser zweiphasigen Mischung gegeben. Je nach Löslichkeit des Substrates wird eine Menge eines organischen Lösungsmittels zugegeben. Diese Mischung wird durch Durchleiten von Stickstoff inertisiert und die Mischung unter starkem Rühren auf die gewünschte Reaktionstemperatur temperiert.

Zu dieser Mischung wird der Katalysator als Lösung in Dichlormethan in Molverhältnissen gegenüber dem Substrat von beispielsweise 1:500 bis 1:5000 gegeben und die Reaktionsmischung die gewünschte Zeit gerührt. Der Umsatz wird chromatographisch verfolgt.

Anschließend kann die Reaktionsmischung nach dem Fachmann bekannten Verfahren aufgearbeitet werden. Es hat sich als vorteilhaft erwiesen, zur Aufarbeitung der Reaktionsmischung Lösungsmittel und verdünnte wässrige Salzsäure oder Wasser zuzusetzen. Nach Phasentrennung kann das Produkt aus der organischen Phase entweder destillativ oder durch ein geeignetes Kristallisationsverfahren in an sich bekannter Weise isoliert werden.

Die erfindungsgemäß herstellbaren 4-Aryl-4-hydroxy-butansäurederivate eignen sich insbesondere für den Einsatz in einem Verfahren zur Herstellung von flüssigkristallinen Verbindungen, Agrochemikalien und Pharmazeutika oder Zwischenprodukten davon.

Bevorzugte Pharmazeutika sind Serotonin-Aufhahmeinhibitoren wie zum Beispiel Fluoxetin und Duloxetin.

Insbesondere eignen sich die erfindungsgemäß herstellbaren 4-Aryl-4-hydroxybutansäurederivate zur Herstellung von Verbindungen der Formeln (VII), (VIII), (IX) und (X),

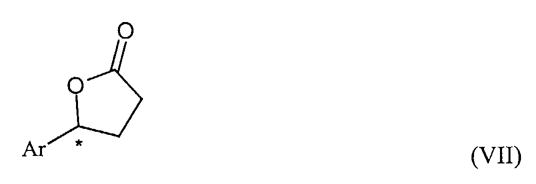

Ar-CH(OH)CH₂CH₂CONR¹ ₙ (VIII),

Ar-CH(OH)CH₂CH₂NR¹ ₙ (IX),

Ar-CH(OH)CH₂CH₂CH₂NR¹ ₙ (X),

in denen jeweils
Ar die unter der Formel (I) genannte Bedeutung besitzt und
wobei in den Formeln (VIII), (IX) und (X) R¹ die in der Formel (I) genannte Bedeutung besitzt und
n = zwei ist.

Die Herstellung von Verbindungen der Formel (VII) kann beispielsweise durch basenkatalysierte Cyclisierung von Verbindungen der Formel (VI) erfolgen, in der W für COOR¹ mit der dort genannten Bedeutung steht. Werden Verbindungen der Formel (VI) eingesetzt, in der W für COOR¹ mit der dort genannten Bedeutung steht, können die Verbindungen der Formel (VII) teilweise auch als ein Reaktionsprodukt des erfindungsgemäßen Verfahrens beobachtet werden, wobei deren Anteil bis zu 20 % üblicherweise jedoch bis zu 10 Mol-% bezogen auf den als Hauptprodukt erhältlichen Hydroxyester betragen kann.

Die Herstellung von Verbindungen der Formel (IX) kann beispielsweise dadurch erfolgen, dass Verbindungen der Formel (VI) gegebenenfalls durch Aminierung in Verbindungen der Formel (VIII) überführt werden und die Verbindungen der Formel (VIII) durch Oxidation mit Hypochlorit zu Verbindungen der Formel (IX) umgesetzt werden.

Die Herstellung von Verbindungen der Formel (X) kann beispielsweise dadurch erfolgen, dass Verbindungen der Formel (VIII) in an sich bekannter Weise mit komplexen Hydriden wie zum Beispiel Lithiumaluminiumhydrid reduziert werden.

Der Vorteil der vorliegenden Erfindung ist, dass 4-Aryl-4-hydroxybutansäurederivate in effizienter und technisch einfach durchführbarer Weise stereoisomerenangereichert erhalten werden können, wobei hohe Katalysatorumsatzzahlen erreicht werden.

### Beispiele

### Allgemeine Arbeitsvorschrift für die Transferhydrierung von 4-Aryl-4-oxobutansäurederivaten

### (Beispiele 1-11)

In einem Schlenkgefäß wird die Katalysatorlösung durch Einwaage von 2,03 Mol-Äquivalenten 1S,2S-N-(p-Toluolsulfonyl)-1,2-diphenylethylen-diamin (S,S-TsDPEN) und 1 Mol-Äquivalent [(Cumol)RuC1₂]2, Rühren dieses Gemisches in 5 ml Dichlormethan und Versetzen mit 2 Mol-Äquivalenten Triethylamin hergestellt.

In einem Mehrhalskolben mit Begasungs-Rührer, Rückflusskühler und Thermometer wird eine Ameisensäure/Triethylamin-Mischung (Molverhältnis 1:1, Molverhältnis 1,05:1 bezogen auf das Substrat) durch langsames Zutropfen innerhalb von 5 min per Tropftrichter von Ameisensäure zum Triethylamin unter Rührung und Eiskühlung hergestellt. Zu diesem zweiphasigen Gemisch wird der entsprechende Ketoverbindung gegeben (100-5000 Äquivalente bezogen auf den Katalysator), die homogene gelbe Lösung gegebenenfalls mit Lösungsmittel versetzt und die Gesamtmischung durch Durchleiten von Argon für 20 min entgast. Es wird auf Solltemperatur temperiert und unter starkem Rühren die dunkelrote Katalysatorlösung auf einmal zum Reaktionsansatz per Spritze geben. Es wird unter Argon für die gegebene Zeit gerührt.

Es wird mit Wasser und Dichlormethan verdünnt, für 10 min nachgerührt, nach Phasentrennung die wässrige Phase 2 mal mit Dichlormethan extrahiert. Die vereinigten organischen Phasen werden mit NaCI-Lösung gewaschen, über MgSO₄ getrocknet, filtriert und dann das Lösungsmittel am Rotationsverdampfer entfernt. Das Rohprodukt wird entweder destilliert, umkristallisiert z.B. aus Hexan/Petrolether bzw. aus Hexan/Dichlormethan oder als Rohmischung in weiteren Reaktionen eingesetzt. Es entsteht üblicherweise ein Gemisch aus Hydroxyestern und Lactonen in Verhältnissen von 99:1 bis 80:20 zugunsten des Hydroxyesters. Die vollständige Umwandlung in die entsprechenden Lactone (siehe Formel VII) erfolgt durch Rühren des Gemisches mit 2 N NaOH-Lösung bei 60°C für 1 h.

Die Umsatz- und Enantiomeren-Analytik erfolgte gaschromatographisch unter Verwendung von Kapillarsäulen der Firma IVA. 12-25 m Säulen vom Typ IVADEX 1 (Säule A), IVADEX 3 (Säule B) und Hydrodex-β-6-TBDM (Säule C) kamen unter Verwendung von Helium als Trägergas auf einem HP 5890 II Gaschromatographen zum Einsatz. Die hier angeführten Umsätze und Reaktionszeiten sind nicht optimiert, da zumeist in geschlossenen Gefäßen ohne Ableitung von CO₂ gearbeitet wurde.

Ethyl-4-hydroxy-4-phenylbutanoat (**1**)
¹H-NMR (d¹-Chloroform, 400 MHz): δ = 7.3-7.1 (m, 5H, Ph), 5.45 (pt, 1H, CHOH), 4.07 (q, 2H, OCH2), 2.36 (t, 2H, CH2), 2.01 (m, 3H, CH2 und OH), 1.18 (t, 3H, CH3) ppm.
ee: 92,0 %, t = 66 h, U = 71,5 %. S/C = 400, T = 30°C.
Chiral-GC: 12,39, 12,92 min (Säule C, 20 m, 160°C isotherm).

Ethyl-4-hydroxy-4-phenylbutanoat (**2**)
t = 24 h, U = 62 %. S/C = 400. T = 30°C.

Methyl-4-hydroxy-4-(4-bromphenyl)butanoat (**3**)
¹H-NMR (d¹-Chloroform, 400 MHz): δ = 7.45 (d, 2H, Ph), 7.14 (d, 2H, Ph), 5.40 (pt, 1H, CHOH), 3.42 (s, 3H, CH3), 2.37 (t, 2H, CH2), 2.20 (br, 1H, OH), 1.98 (pq, 2H, CH2) ppm.
ee: 89,9 %, t = 66 h, U = 96 %. S/C = 400, T = 30°C.
Chiral-GC: Ester: 18,98, 19,64 min, Lacton: 16,20, 17,24 min (Säule A, 12.5 m, 15 min 160°C, 2°C/min, 220°C).

Methyl-4-hydroxy-4-(4-bromphenyl)butanoat (**4**)
t = 24 h, U = 95 %. S/C = 100, T = 30 °C.

Methyl-4-hydroxy-4-(4-methoxyphenyl)butanoat (**5**)
ee: 91,8 %, S/C = 400, T = 30°C.
Chiral-GC: Lacton: 20,08, 20,41 min (Säule A, 12,5 m, 15 min 145°C, 5°C/min, 180°C).

Ethyl-4-hydroxy-4-(2-thienyl)butanoat (**6**)
¹H-NMR (d¹-Chloroform, 400 MHz): δ = 7.17 (d, 1H, Ar), 6.89 (m, 3H, Ar), 4.95 (pt, 1H, CHOH), 4.09 (q, 2H, OCH2), 2.40 (m, 3H, CH2 und OH), 2.01 (pq, 2H, CH2), 1.19 (t, 3H, CH3) ppm.
ee: 96,00 %, S/C = 500, U = 98 %, t = 60 h, T = 30°C.
Chiral-GC: Hydroxyester: 13,30, 13,63; Lacton: 6,77, 7,04 min. (Säule A, 12,5 m, 15 min 145°C, 5°C/min, 180°C).

Ethyl-4-hydroxy-4-(2-thienyl)butanoat (**7**)
ee: 94,8 %, S/C = 100, U = 91,7 %, t = 18 h, T = 30 °C.

Methyl-4-hydroxy-4-(2,4-dichlorothien-3-yl)butanoat (**8**)
¹H-NMR (d¹-Chloroform, 400 MHz): δ = 6.82 (s, 1H, Ar), 4.81 (dd, 1H, CHOH), 3.62 (s, 3H, CH3), 2.39 (t, 2H, CH2), 2.20 (br, 3H, OH und CH2) ppm.
ee: 70,00 %, S/C = 200, U = 36,5 %, t = 66 h, T = 30°C.
Chiral-GC: Hydroxyester: 21.62, 22.08; Lacton: 18.02, 18.44 min. (Säule A, 12.5 m, 15 min 145°C, 5°C/min, 180°C).

Methyl-4-hydroxy-4-(2,4-dichlorothien-3-yl)butanoat (**9**)
S/C = 100, U = 97 %, t = 24 h, T = 30°C.

Ethyl-4-hydroxy-4-(2-thienyl)butanoat (**10**)
Die Umsetzung erfolgt analog Beispiel 6, außer dass 0.004 eq. [N-[(1R,2R und 1S,2S)-2-(amino-κN)-1,2-diphenylethyl]-p-tolylsulfonamidato-κN]chloro[(rib)-1,3,5-trimethylbenzol]-ruthenium(II) als Katalysator eingesetzt wurden.
ee: 94,8 %, S/C = 250, U = 80 %, t = 26 h, T = 30°C.

Ethyl-4-hydroxy-4-(2-thienyl)butanoat (**11**)
Die Umsetzung erfolgt analog Beispiel 6, außer dass 0.002 eq. [N-[(1R,2R und 1S,2S)-2-(amino-κN)-1,2-diphenylethyl]-p-tolylsulfonamidato-κN]chloro[(η⁶)-1,3,5-trimethylbenzol]-ruthenium(II) als Katalysator eingesetzt wurden.
ee: 94,3 %, S/C = 500, U >98 %, t = 60 h, T = 30°C.

## Patentansprüche

1. Verfahren zur Herstellung von stereoisomerenangereicherten 4-Aryl-4-hydroxybutansäurederivaten, **dadurch gekennzeichnet, dass**
a) Verbindungen der Formel (I)
Aryl-CO-CH₂CH₂W (I),
in der
Aryl für einen mono-, bi- oder tricyclischen aromatischen Rest mit insgesamt 5 bis 18 Ringatomen steht, wobei pro Cyclus keines, eines zwei oder drei Ringatome ausgewählt aus der Gruppe Sauerstoff, Schwefel und Stickstoff vorhanden sein können, und wobei der mono-, bi- oder tricyclische aromatische Rest gegebenenfalls einfach oder mehrfach substituiert ist und
W für C(O)YR¹ₙ steht, wobei Y für Sauerstoff steht und n = 1 ist oder Y für Stickstoff steht und n = 2 ist, oder
W für CN steht und,
R¹ jeweils unabhängig voneinander für Wasserstoff, C₁-C₂₀-Alkyl, C₄-C₁₄-Aryl oder C₅-C₁₅-Arylalkyl oder im Falle, dass Y für Stickstoff steht, die beiden Reste R¹ zusammen für C₃-C₁₂-Alkylen stehen,
b) in Gegenwart eines Ruthenium enthaltenden Katalysators und
c) in Gegenwart von mindestens einem Amin, das zumindest teilweise in protonierter Form vorliegt,
d) mit Ameisensäure, Formiaten oder Mischungen davon
umgesetzt werden.

2. Verfahren nach Anspruch l, **dadurch gekennzeichnet, dass** es in Gegenwart von organischem Lösungsmittel durchgeführt wird.

3. Verfahren nach einem oder mehreren der Ansprüche 1 bis 2, **dadurch gekennzeichnet, dass** in Formel (I) Aryl steht für einen mono- oder bicyclischen aromatischen Rest mit insgesamt 5 bis 12 Ringatomen, wobei pro Cyclus keines, eines oder zwei Ringatome ausgewählt sind aus der Gruppe Sauerstoff, Schwefel und Stickstoff und wobei der mono- oder bicyclische aromatische Rest keinen, einen, zwei oder drei Reste pro Cyclus trägt, die jeweils unabhängig voneinander ausgewählt sind aus der Gruppe Hydroxy, C₁-C₁₂-Alkyl, Cyano, COOH, COOM, COO-(C₁-C₁₂-Alkyl), COO-(C₄-C₁₀-Aryl). CO-(C₁-C₁₂-Alkyl), CO-(C₄-C₁₀-Aryl), O-(C₁-C₁₂-Alkyl), (C₁-C₁₂-Alkyl)-O-(C₁-C₁₂-Alkyl), (C₄-C₁₀-Aryl)-O-(C₁-C₁₂-Alkyl), O-(C₄-C₁₀-Aryl), O-CO-(C₄-C₁₀-Aryl), O-CO-(C₁-C₁₂-Alkyl), OCOO-(C₁-C₁₂-Alkyl), N-(C₁-C₁₂-Alkyl)₂, NH-(C₁-C₁₂-Alkyl), N(C₄-C₁₀-Aryl)₂, NH-(C₄-C₁₀-Aryl), Fluor, Chlor, Brom, Iod, NO₂, SO₃H, SO₃M, SO₂(C₁-C₁₂-Alkyl), SO(C₁-C₁₂-Alkyl), C₁-C₁₂-Fluoralkyl, wobei Fluoralkyl für einen einfach, mehrfach oder vollständig durch Fluor substituierten Alkylrest gemäß obiger Definition steht, NHCO-(C₁-C₁₂-Alkyl), CONH₂, CONH-(Cᵢ-Cᵢ₂-Alkyl), NHCOO-(Ci-Ci2-Alkyl), PO(C₄-Cᵢₒ-Aryl)₂, PO(C₁-C₁₂-Alkyl)₂, PO₃H₂, PO₃M_{2,} POßHM_{,} PO(0(Ci-Cᵢ₂-Alkyl)₂, wobei M jeweils für ein Alkalimetallion oder ein halbes Äquivalent eines Erdalkalimetallions steht.

4. Verfahren nach einem oder mehreren der Ansprüche 1 bis 3, **dadurch gekennzeichnet, dass** in Formel (I) W für COOR¹ steht, wobei R¹ für Wasserstoff oder C₁-C₈-Alkyl steht.

5. Verfahren nach einem oder mehreren der Ansprüche 1 bis 4, **dadurch gekennzeichnet, dass** als Verbindung der Formel (I) Methyl-4-oxo-4-(2-thiophenyl)butanoat oder Ethyl-4-oxo-4-(2-thiophenyl)butanoat eingesetzt werden.

6. Verfahren nach einem oder mehreren der Ansprüche 1 bis 5, **dadurch gekennzeichnet, dass** Katalysatoren verwendet werden, die Rutheniumkomplexe enthalten, die durch Umsetzung von Verbindungen der Formel (II) mit Verbindungen der Formel (III) erhältlich sind, oder Komplexe der Formel (IV), wobei
in den Verbindungen der Formel (II)
[RuX₂(Aren)]₂ (II)
Aren steht für eine koordinierte aromatische Verbindung mit 6 bis 12 Ringkohlenstoffatomen, die weiterhin mit bis zu 6 Resten substituiert sein kann, die jeweils unabhängig voneinander ausgewählt sind aus der Gruppe C₁-C₈-Alkyl, Benzyl und Phenyl und
X für Chlor, Brom oder Iod steht und wobei
in der Formel (III) 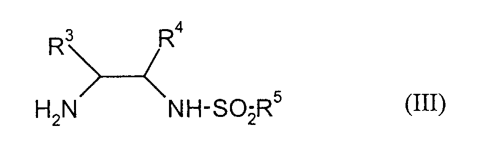
R³ und R⁴ jeweils unabhängig voneinander beispielsweise für C₁^{-C}₂₀-Alkyl, C₄-C₁₅-Aryl oder C₅-C₁₆-Arylalkyl oder R³ und R⁴ zusammen für einen geradkettigen oder verzweigten C₃-C₁₂-Alkylen-Rest, und
R⁵ für C₁-C₂₀-Alkyl, C₁-C₂₀-Fluoralkyl oder C₄-C₁₅-Aryl stehen und wobei
in der Formel (IV)
[RuX₂(aren){(III)}] (IV)
Aren und X jeweils die unter Formel (II) angegebene Bedeutung besitzt und (III) für Verbindungen der Formel (III) mit der dort angegebenen Bedeutung steht.

7. Verfahren nach einem oder mehreren der Ansprüche 1 bis 6, **dadurch gekennzeichnet, dass** als Katalysator solche verwendet werden, die Rutheniumkomplexe enthalten, die durch Umsetzung von S,S- oder R.R-N-p-Toluolsulfonyl- 1 ,2-diphenylethylendiamin und Cumoldichlororuthenium-Dimer erhältlich sind.

8. Verfahren nach einem oder mehreren der Ansprüche 1 bis 7, **dadurch gekennzeichnet, dass** Mischungen von Ameisensäure mit Aminen eingesetzt werden.

9. Verfahren nach einem oder mehreren der Ansprüche 1 bis 8, **dadurch gekennzeichnet, dass** als Amine solche der Formel (V) eingesetzt werden,
NR⁶R⁷R⁸ (V)
in der
R⁶, R⁷ und R⁸ jeweils unabhängig voneinander für Wasserstoff, C₁-C₈-Alkyl oder Benzyl stehen.

10. Verfahren nach einem oder mehreren der Ansprüche 1 bis 9, **dadurch gekennzeichnet, dass** als Amin Triethylamin eingesetzt wird.

11. Verfahren nach einem oder mehreren der Ansprüche 1 bis 10, **dadurch gekennzeichnet, dass** das molare Verhältnis von Ameisensäure zu Amin 1:1 bis 3:1 beträgt.

12. Verfahren nach einem oder mehreren der Ansprüche 1 bis 11, **dadurch gekennzeichnet, dass** das molare Verhältnis an Ameisensäure bezogen auf eingesetztes Substrat 1:1 bis 3:1 beträgt.

13. Verfahren nach einem oder mehreren der Ansprüche 1 bis 12, **dadurch gekennzeichnet, dass** die Reaktionstemperatur -10 bis 150°C beträgt.

14. Verfahren nach einem oder mehreren der Ansprüche 1 bis 13, **dadurch gekennzeichnet, dass** die molare Menge an Ruthenium 0,01 bis 1,0 Mol-%, bezogen auf das eingesetzte Substrat beträgt.

15. Verwendung von stereoisomerenangereicherten 4-Aryl-4-hydroxy-butansäurederivaten, die nach einem oder mehreren der Ansprüche 1 bis 14 hergestellt wurden zur Herstellung von flüssigkristallinen Verbindungen, Agrarchemikalien, Pharmazeutika oder Zwischenprodukten davon.

16. Verwendung nach Anspruch 15, **dadurch gekennzeichnet, dass** die Pharmazeutika Serotonin-Aufnahmeinhibitoren sind.

17. Verwendung nach Anspruch 16, **dadurch gekennzeichnet, dass** die Serotonin-Aufnahmeinhibitoren Fluoxetin oder Duloxetin sind.

18. Verwendung von stereoisomerenangereicherten 4-Aryl-4-hydroxy-butansäurederivaten, die nach einem oder mehreren der Ansprüche 1 bis 14 hergestellt wurden zur Herstellung von Verbindungen der Formeln (VII), (VIII), (IX) und (X), 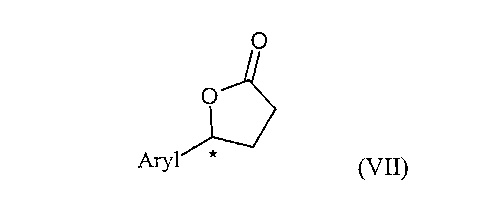
Aryl-CH(OH)CH₂CH₂CONR¹ ₙ (VIII),
Aryl-CH(OH)CH₂CH₂NR¹ ₙ (IX),
Aryl-CH(OH)CH₂CH₂CH₂¹ ₙ (X),
in denen jeweils
Aryl die unter der Formel (I) im Anspruch 1 genannte Bedeutung besitzt und wobei in den Formeln (VIII), (IX) und (X) R¹ die in der Formel (I) im Anspruch 1 genannte Bedeutung besitzt und
n = zwei ist.
